(19) Europäisches Patentamt
European Patent Office
Office européen des brevets

(11) Veröffentlichungsnummer: **0 079 397**
**A1**

(12) **EUROPÄISCHE PATENTANMELDUNG**

(21) Anmeldenummer: 81109668.4

(22) Anmeldetag: 13.11.81

(51) Int. Cl.³: C 07 C 103/46
A 61 K 49/04

(43) Veröffentlichungstag der Anmeldung:
25.05.83 Patentblatt 83/21

(84) Benannte Vertragsstaaten:
AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Pfeiffer, Heinrich, Dr.
Pulfrichzeile 9
D-1000 Berlin 20(DE)

(72) Erfinder: Mützel, Wolfgang, Dr.
Weddigenweg 74
D-1000 Berlin 45(DE)

(72) Erfinder: Speck, Ulrich, Dr.
Benediktiner Strasse 50
D-1000 Berlin 28(DE)

(54) **Unsymmetrisch substituierte Dicarbonsäure-bis-(2,4,-6-trijodanilide), deren Herstellung und diese enthaltende Röntgenkontrastmittel.**

(57) Unsymmetrisch substituierte Dicarbonsäure-bis-(2,4,6-trijodanilide) der Formel I

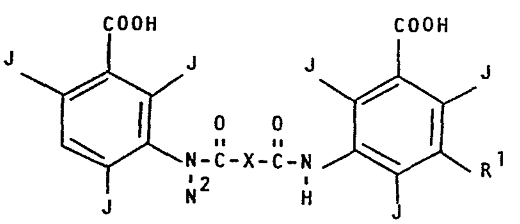

(I),

worin

X ein gerad- oder verzweigtkettiges Alkylen, das durch eine oder mehrere Sauerstoffatome unterbrochen sein kann,

$R^1$ den Rest $-NHAcyl$, $-CH_2NHAcyl$ oder $-CONHR^3$ bedeutet, wobei $R^3$ Wasserstoff oder einen niederen, gerad- oder verzweigtkettigen, gegebenenfalls mono- oder polyhydroxylierten Alkylrest darstellt und vorhandene Hydroxylgruppen funktionell abgewandelt vorliegen können

und

$R^2$ Wasserstoff oder einen niederen Alkylrest bedeuten.

Die verbindungen der Formel I sind aufgrund ihrer guten pharmakologischen und physikochemischen Eigenschaften auf allen Anwendungsgebieten von wasserlöslichen Röntgenkontrastmitteln, insbesondere für die i.v. Cholegraphie und für die Computertomographie hervorragend geeignet.

EP 0 079 397 A1

COMPLETE DOCUMENT

Croydon Printing Company Ltd.

Die Erfindung betrifft Unsymmetrisch substituierte Dicarbonsäure-bis-(2,4,6-trijodanilide) der Formel I

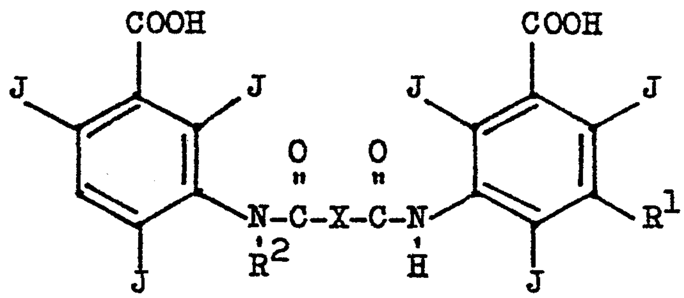

(I),

worin

X ein gerad- oder verzweigtkettiges Alkylen, das durch ein oder mehrere Sauerstoffatome unterbrochen ist,

$R^1$ den Rest -NHAcyl, $-CH_2NHAcyl$ oder $-CONHR^3$ bedeutet, wobei $R^3$ Wasserstoff oder einen niederen, gerad- oder verzweigtkettigen, gegebenenfalls mono- oder polyhydroxylierten Alkylrest darstellt und vorhandene Hydroxylgruppen funktionell abgewandelt vorliegen können

und

$R^2$ Wasserstoff oder einen niederen Alkylrest bedeuten,

deren physiologisch verträgliche Salze mit anorganischen oder organischen Basen,
sowie ein Verfahren zu deren Herstellung und neue Röntgenkontrastmittel, die Verbindungen der Formel I als schattengebende Substanz enthalten.

X als gerad- oder verzweigtkettiges Alkylen, das durch ein oder mehrere Sauerstoffatome unterbrochen ist, kann 1 bis 12, vorzugsweise 1 bis 10 Kohlenstoffatome enthalten.

Insbesondere geeignet ist ein geradkettiges Alkylen mit 1 bis 8 Kohlenstoffatomen, das durch 1 bis 4, vorzugsweise durch 1 bis 3 Sauerstoffatome unterbrochen sein kann.
Als Beispiele seien hier genannt

$-CH_2-$, $-(CH_2)_2-$, $-(CH_2)_3-$, $-(CH_2)_4-$, $-(CH_2)_6-$, $-(CH_2)_8-$,
$-CH_2-O-CH_2-$, $-(CH_2OCH_2)_2$ und $-(CH_2-O-CH_2)_3-$.

Als verzweigtkettige Reste X kommen infrage:

$-HC(CH_3)-$, $-C(CH_3)_2-$, $-CH_2-CHCH_3-(CH_2)_2-$, $-CH_2-CHCH_3-CH_2-$
und ähnliche.

Bedeutet $R^1$ den Rest -NHAcyl oder $-CH_2NHAcyl$, so leitet sich der Acylrest von einer aliphatischen Carbonsäure mit 2 bis 4 Kohlenstoffatomen ab. Geeignet sind insbesondere aliphatische Carbonsäurereste mit 2 bis 3 Kohlenstoffatomen wie beispielsweise der Propionylrest und vorzugsweise der Acetylrest.

Bevorzugt geeignet sind Acylreste, die durch

1 bis 2 Hydroxylgruppen, vorzugsweise durch eine Hydroxylgruppe substituiert sind. Beispielsweise genannt seien der Hydroxyacetyl- und der α-Hydroxypropionylrest.

Der Rest $R_2$ als niederer Alkylrest enthält 1 bis 4, vorzugsweise 1 bis 3 und insbesondere 1 bis 2 Kohlenstoffatome. Beispielsweise genannt seien insbesondere der Methyl- und Äthylrest;
bevorzugt ist der Methylrest.

Ist der Alkylrest $R_3$ unsubstituiert, enthält er 1 bis 4, vorzugsweise 1 bis 3 und insbesondere 1 bis 2 Kohlenstoffatome. Beispielsweise genannt seien der Methyl- und der Äthylrest.

Ist der Alkylrest $R_3$ ein Mono- oder Polyhydroxyalkylrest, kann er gerad- oder verzweigtkettig sein.
Bevorzugt geeignet sind Alkylreste mit 2 bis 5 Kohlenstoffatomen, vorzugsweise mit 2 bis 4 Kohlenstoffatomen. Die Hydroxylgruppen im Alkylrest können als primäre und/oder sekundäre und/oder tertiäre Hydroxylgruppen vorliegen. Der Alkylrest kann 1 bis 4, vorzugsweise 1 bis 3 Hydroxylgruppen enthalten.
Beispielsweise genannt seien der Hydroxyäthyl- und der 1,3-Dihydroxy-propyl-(2)-rest.

Liegen die Hydroxylgruppen im Acylrest und im Rest $R^3$ in funktionell abgewandelter Form vor, so handelt es sich um Äthergruppen, beispielsweise um die Äthoxy- und die Methoxygruppe; vorzugsweise genannt sei die Methoxygruppe.

Sollen die Verbindungen der Formel I in Form ihrer physiologisch verträglichen Salze angewendet werden, so kommen zur Salzbildung die dem Fachmann dafür bekannten sowohl anorganischen als auch organischen Basen infrage. Die Herstellung der Salze erfolgt durch Umsetzung der entsprechenden Säure mit der Base in an sich bekannter Weise.

Als physiologisch verträgliche Salze mit Basen kommen somit sowohl Metallsalze, wie zum Beispiel Natrium-, Calcium und Magnesiumsalze, infrage als auch Aminsalze, wie beispielsweise Glucamin-, N-Methylglucamin-, N,N-Dimethylglucamin-, Äthanolamin-, Diäthanolamin-, Morpholinsalze u.a..
Zur Salzbildung geeignet sind auch basische Aminosäuren und Aminosäureamide.

Zur röntgenographischen Darstellung der Gallengänge und der Gallenblase sind Kontrastmittel erforderlich, die über Leber und Galle ausgeschieden werden.
Alle intravenös zu verabreichenden Cholegraphika, die sich klinisch bewährt haben, besitzen die erstmals im Iodipamid (Formel IVa) (DPP 936 928/926 545) verwirklichte Struktur IV

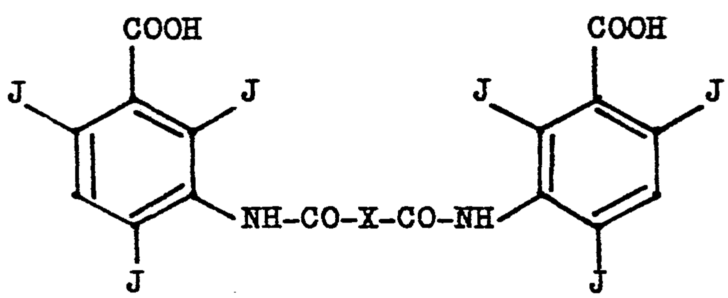

(IV)

(Iodipamid IV a : $X = -(CH_2)_4-$) .

Deren Strukturmerkmale sind: 2 Trijodierte Aniline mit je einer freien Carboxylgruppe und mit je einem Wasserstoffatom in 5-Stellung, sowie eine die beiden aromatischen Kerne verbindende Dicarbonsäure. Von diesen Strukturmerkmalen wurde bisher in den handelsüblichen Cholegraphika einerseits das Brückenglied X variiert und andererseits der Wasserstoff in 5- und 5'Stellung substituiert.

Entsprechende Untersuchungen mit diesen und ähnlichen Verbindungen (s.a. Röntgenkontrastmittel v.R. Barke, Georg Thieme Verlag Leipzig 1970, S. 102 ff.) führten zu der Erkenntnis, daß jedes der genannten Strukturelemente seine Bedeutung für die Ausscheidung über die Galle hat. Insbesondere die in 5,5'-Stellung befindliche unsubstituierte Wasserstoffatome scheinen essentiell für die Bindung an Plasmaeiweißstoffe und Transportproteine zu sein.

Denn die Erfahrung hat gezeigt, daß Kontrastmittel der Formel IV, die in 5,5'-Stellung durch Acylamino-oder Aminocarbonylgruppen substituiert sind, zwar etwas besser verträglich sein können, dafür aber erheblich weniger gallengängig sind und verstärkt über die Niere ausgeschieden werden (s. DOS 1 618 001, DOS 2 422 718).

Der vorliegenden Erfindung lag die Aufgabe zugrunde, neue schattengebende Substanzen zu entwickeln, die neben guter Gallengängigkeit auch eine bessere Verträglichkeit aufweisen.

Durch die Herstellung der erfindungsgemäßen Verbindungen der Formel I, bei denen nur eines der beiden 5,5'-ständigen Wasserstoffatome substituiert ist, wurden neue schattengebende Substanzen erhalten, die zwar deutlich hydrophiler und damit besser verträglich als ihre vergleichbaren in 5- und 5'-Stellung unsubstituierten Ausgangsverbindungen aber dennoch ausreichend an Plasmaproteine gebunden sind und ganz überwiegend über die Galle ausgeschieden werden. Dies zeigt ein Vergleich der Werte für Proteinbindung, Verträglichkeit, biliäre und renale Ausscheidung der Megluminsalze von Iodipamid (B) und Hexandisäure-1-(3-carboxy-2,4,6-trijod-anilid)-6-(3-carboxy-5-N-methylcarbamoyl-2,4,6-trijodanilid) (A) gezeigt wird (Tabelle 1):

**Tabelle I**

| Verbindung | A | B |
|---|---|---|
| Verteilungskoeffizient Butanol/Wasser pH 7.4 | 0,083 ± 0,001 | 0,278 ± 0,008 |
| Proteinbindung an Humanserum bei ca. 1,2 mg Jod/ml | 43 % | 77 % |
| 1) $DL_{50}$, i.v., Ratte (g Jod/kg) | 5,5 | 2,6 |
| 2) Ratte, pericerebral, $ED_{50}$ (mg Jod/kg) | 17,1 (11,8-23,6) | 6,35 (5,11-7,44) |
| 3) Ratte, intrazisternal, $ED_{50}$ (mg Jod/kg) | 15,6 (11,5-21,1) | 1,07 (0,54-1,83) |
| 4) biliäre Ausscheidung (% der Dosis) | | |
| bis 30 min. | 61,3 ± 7,8 | 59,4 ± 3,6 |
| bis 3 h p.inj. | 85,9 ± 3,0 | 91,5 ± 2,3 |
| 5) renale Ausscheidung (% der Dosis) bis 3 h p.inj. | 22,9 ± 3,3 | 10,8 ± 2,0 |

Erläuterungen zu Tabelle 1:

1) Injektion der Kontrastmittel als Megluminsalzlösungen mit 180 mg Jod/ml mit einer Geschwindigkeit von 0,8 ml/min bei 100 g schweren männlichen und weiblichen Ratten.

2) Männlichen und weiblichen Ratten im Gewicht von etwa 100 g wurden in leichter Äthernarkose je 0,04 ml unterschiedlich konzentrierter Kontrastmittellösungen nach der Methode von Valzelli durch eine natürliche Knochenspalte des Schädels zwischen Joch- und Hinterhauptbein intrazerebral injiziert. Die Injektion erfolgte mit einer Hamilton Spritze 710 N, bei der die Einstichtiefe auf 10 mm begrenzt ist. Je Präparat wurden mindestens 4 Dosierungen an 10 Tieren untersucht. Als Wirkungskriterien wurden über den Zeitraum von 24 Stunden Exzitationszustände, schwere Haltungsanomalien, Koma und Tod bewertet.

3) Bei grundsätzlich gleicher Versuchsanlage wie unter 2) beschrieben, wurden 200-250 g schweren Ratten die Kontrastmittel in die Cisterna suboccipitalis injiziert. Die Kontrastmittel wurden mit 3%iger Mannit-Lösung verdünnt, um den osmotischen Druck anzupassen.

4) Ratten (ca. 150 g Körpergewicht) wurde in Äthernarkose ein Polyäthylenschlauch in den Ductus choledochus eingebunden. Etwa eine Stunde nachdem die Tiere aus der Narkose erwacht waren, wurden die Kontrastmittel in einer Dosis von 60 mg Jod/kg intravenös injiziert und die Galle bis 3 Stunden nach der Injektion fraktioniert gesammelt.

5) Intakten Ratten von etwa 100 g Körpergewicht wurde 20 Minuten vor der Verabreichung des Kontrastmittels 5 ml Wasser und dann in 1-stündigem Abstand nach der Verabreichung der Prüfsubstanzen 3 mal je 2 ml Wasser

oral verabreicht. bis 3 Stunden nach der i.v. Injektion des Kontrastmittels in einer Dosis von 60 mg Jod/kg wurde der Harn quantitativ gesammelt.

4), 5) die Messung des Kontrastmittels erfolgte entweder aufgrund der $^{131}$J-Aktivität in den Proben oder bei nicht radioaktiv markierten Kontrastmitteln mit Hilfe der Röntgenfluoreszenz des Jods.

Überraschenderweise zeigen die unsymmetrisch substituierten erfindungsgemäßen Verbindungen der Formel I außerdem eine außerordentlich starke Anreicherung im Leberparenchym von Versuchstieren (Hund und Affe), wodurch die Verbindungen der Formel I für die Computer-Tomographie hervorragend geeignet sind.

Die Computertomographie (CT) ermöglicht die bildhafte Darstellung von Schnitten durch den lebenden Organismus aufgrund der unterschiedlichen Absorption von Röntgenstrahlen in Körperflüssigkeiten, Organen und Geweben. Anders als bei der konventionellen Radiologie wird in der CT eine quantitative Messung der Röntgenstrahlen durchgeführt und ausgewertet. Dies Verfahren erlaubt eine Unterscheidung von Strukturen mit sehr geringen Absorptionsuntersunterschieden.

Dennoch finden sich im menschlichen Köŕper Gewebe und pathalogisch veränderte Organe, die für eine Darstellung selbst mit Hilfe der CT keine ausreichenden Absorptionsunterschiede aufweisen. Ein wichtiges Problem in dieser Hinsicht sind die im Parenchym der Leber vorhandenen Läsionen wie Tumore, Metastasen, etc. Man hat daher versucht, wie in der konventionellen Radiologie, durch Verabreichung von jodhaltigen Kontrastmitteln eine

bessere Unterscheidung des pathologischen Gewebes von dem gesunden Parenchym zu erzielen. Tatsächlich gelingt in einigen Fällen durch Verabreichung eines Urographicums eine solche Abgrenzung. Die unterschiedliche Anreicherung des Kontrastmittels in gesundem und pathologischem Gewebe hält jedoch nur sehr kurze Zeit an und ist wenig ausgeprägt, da sich das Kontrastmittel nur unspezifisch im intravasalen und interstitiellen Raum der Leber verteilt. Es kann sogar zur Abschwächung nativ vorhandener Absorptionsdifferenzen der Gewebe kommen (Fuchs, W.A., P. Vock und M. Haertel: Pharmakokinetik intravasaler Kontrastmittel bei der Computer-Tomographie; Radiologe 19, 90-93, 1979). Die Verwendung von Gallenkontrastmitteln für die Darstellung der Leber war zwar naheliegend, hat jedoch zu keiner Anreicherung in den Leberzellen geführt (Hübener, K.H.: Computertomographische Densitometrie von Leber, Milz und Nieren bei intravenös verabreichten lebergängigen Kontrastmitteln in Bolusform; Fortschr. Röntgenstr. 129, 289-307, 1978). Andere Untersucher haben Partikel in Form von Emulsionen (Lamarque, J.L. et al.: The use of iodolipids in hepatosplenic computed tomography; J. Comput. Assist. Tomogr. 3, 21-24, 1979), Suspensionen (Violante, M.R., P.B. Dean: Improved Detectability of VX2 Carcinoma in the Rabbit Liver with Contrast Enhancement in Computed Tomography; Radiology 134, 237-239, 1980) oder Liposomen intravenös verabreicht. Mit derartigen Kontrastmitteln wurde eine sehr befriedigende Dichteanhebung des gesunden Leberparenchyms erzielt und eine gute Differenzierung des pathologischen Gewebes. Der routinemäßigen klinischenAnwendung steht allerdings die oft hohe Toxizität und/oder die unsichere Stabilität der Zubereitungen im Wege. Als wasserlösliches Kontrastmittel für eine Kontrastverstärkung des Leberparenchyms wurde bisher nur die Iosefamsäure beschrieben (Köhler, R.E., R.J. Stanley, R.G. Evens: Iosefamate Meglumine:

An Iodinated Contrast Agent for Hepatic Computed Tomography Scanning; Radiology 132, 115-118, 1979). Die Iosefamsäure erreicht beim Hund eine noch ausreichende, beim Menschen jedoch für eine Verbesserung der Diagnostik nicht mehr genügende Konzentration in der Leber.

Die neuen erfindungsgemäßen Verbindungen der Formel I liefern dagegen schon bei Infusion einer verhältnismäßig geringen Kontrastmitteldosis und relativ niedrigem Blutspiegel beim Hund eine starke Anreicherung des Kontrastmittels in der Leber, wie sie für die Computertomographie gefordert wird. Darüber hinaus liefern die erfindungsgemäßen Verbindungen der Formel I hochkonzentrierte wässrige Salzlösungen, die hitzesterilisierbar und langfristig lagerungsstabil sind.

Die außerordentlich starke Anreicherung der erfindungsgemäßen Verbindungen der Formel I geht aus Tabelle 2 hervor, wo die maximale Anreicherung in der Leber nach intravenöser Verabreichung der Megluminsalzlösungen von Iosefamsäure (D) und von 3-Oxapentandisäure-1-(3-carboxy-2,4,6-trijod-N-methylanilid)-5-(3-carboxy-5-N-methylcarbamoyl-2,4,6-trijodanilid) (E) zusammengestellt wurde.

0079397

Tabelle 2

| Verbindung | maximale Anreicherung in der Leber des Hundes | |
|---|---|---|
| | 360 mg Jod/kg EMI-Units | min. p. appl. |
| D | 22,8 | 80 |
| E | 42,1 | 90 |

Die Werte wurden an gesunden weiblichen und männlichen Beagle-Hunden (Züchter: Schering) im Gewicht von 9-14 kg ermittelt.

Die Megluminsalzlösungen enthielten 180 mg Jod/ml.

Die Untersuchungen zur Röntgendichte der Leber vor und alle 5 Minuten bis 90 Minuten nach Gabe der Kontrastmittel wurden mit einem Ganzkörper-Scanner vom Typ EMI CT 5005/12 durchgeführt, der nach dem Rotations-Translations-Prinzip arbeitet. Die Scanzeit betrug 20 Sekunden, die Röhrenspannung 120 kV. Bei einer Bildmatrix von 320 x 320 Elementen betrug die Standardabweichung vom Mittelwert eines normierten Wasserphantoms 1,6 % (d.h. ± 8 EU = EMI-Units.

Die Auswertung der Computertomogramme stets des gleichen Leberareals erfolgte mit Hilfe der Auswerteeinheit "IVC" der Fa. EMI.

Die erfindungsgemäßen Verbindungen sind aber nicht nur für die Cholegraphie und für die Computer-Tomographie geeignet, sie sind auch als schattengebende Substanzen auf verschiedenen Anwendungsgebieten von wasserlöslichen Röntgenkontrastmitteln, insbesondere zur Darstellung von Körperhöhlen und auch z.B. der ableitenden Harnwege des Magen-Darm-Kanals, der Gelenkhöhlen, des tracheobronchialen Systems und in der Histerosalpingographie grundsätzlich geeignet.

In allen Fällen kommt es durch die neuen Röntgenkontrastmittel zu einer besonders guten Detail-Erkennbarkeit der dargestellten Strukturen.

Die Erfindung betrifft somit auch neue Röntgenkontrastmittel auf Basis von Verbindungen der allgemeinen Formel I.

Die Herstellung der neuen Röntgenkontrastmittel auf Basis der erfindungsgemäßen Verbindungen der allgemeinen Formel I erfolgt nach an sich bekannten Methoden, z.B. dadurch, daß man die schattengebende Substanz mit den in der Galenik üblichen Zusätzen, z.B. Stabilisatoren wie Natriumedetat, Calcium-di-natriumedetat, physiologisch verträglichen Puffern, wie beispielsweise Trometamin-Puffer u.ä., in eine für die intravenöse Applikation geeignete Form bringt. Die Konzentration der neuen Röntgenkontrastmittel im wäßrigen Medium richtet sich nach der röntgendiagnostischen Methode. Die bevorzugten Konzentrationen und Dosierungen der neuen Verbindungen bewegen sich in den Bereichen von 50-400 mg J/ml für die Konzentrationen und 5-500 ml für die Dosierung. Besonders bevorzugt sind Konzentrationen zwischen 100-400 mg J/ml.

Das Verfahren zur Herstellung der neuen Verbindungen der Formel I ist dadurch gekennzeichnet, daß man eine Verbindung der allgemeinen Formel II

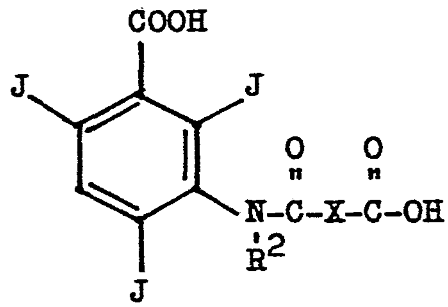

(II) ,

worin $R^2$ und X die obengenannte Bedeutung hat, mit einem Chlorierungsreagenz und einem trijodierten Aminobenzoesäure der allgemeinen Formel III

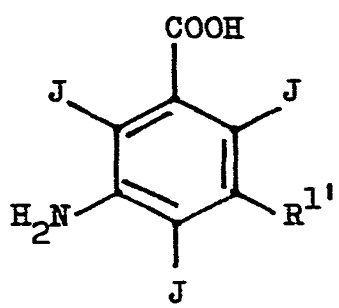

(III) ,

worin $R^{1'}$ die obengenannte Bedeutung hat, umsetzt.

Die Amidierungsreaktion erfolgt in einem geeigneten polaren Lösungsmittel wie beispielsweise Dioxan, Dimethylformamid oder Dimethylacetamid oder deren Gemischen in an sich bekannter Weise, dadurch daß eine Verbindung der Formel II zunächst bei 0°C bis 40°C, vorzugsweise 0°C bis 20°C, mit einem Chlorierungsreagenz zum Säurechlorid der allgemeinen Formel V

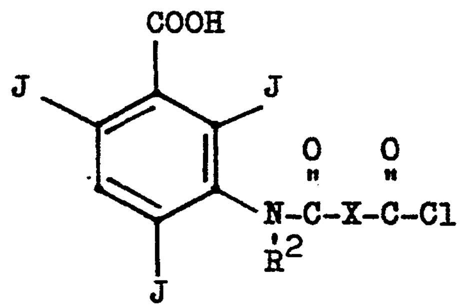

( V) ,

mit $R^2$ und X in der oben genannten Bedeutung umgesetzt wird, das danach ohne Isolierung durch Umsetzung mit einer Verbindung der allgemeinen Formel III bei einer Temperatur von 0°C bis 80°C, vorzugsweise bei 20°C bis 40°C, die erfindungsgemäßen Verbindungen der allgemeinen Formel I liefert.

Als Chlorierungsreagenzien sind beispielsweise Phosphorpentachlorid Oxalylchlorid, Phosgen oder 1,1-Dichlormethylmethylenäther, vorzugsweise Thionylchlorid geeignet.

Nach beendeter Umsetzung wird die Verbindung der Formel I durch Wasserfällung isoliert und durch Salzbildung mit einer geeigneten anorganischen oder organischen Base wie beispielsweise Ammoniak oder einem Amin oder Alkalihydroxid nach dem Fachmann bekannten Verfahren gereinigt.

Der intermediäre Schutz von freien Hydroxylgruppen erfolgt nach üblichen Methoden durch leicht wieder abspaltbare Schutzgruppen. Die Einführung solcher Gruppen kann durch Acylierung (z.B. Einführung eines vorzugsweisen Acetylrestes oder Benzoylrestes), durch Verätherung (z.B. Einführung des Triphenylmethylrestes) oder durch Acetalisierung oder Ketalisierung z.B. mittels Acetaldehyd, Dihydropyran, Aceton oder 2,2-Dimethoxypropan erreicht werden.

Die spätere Abspaltung der intermediär eingeführten Schutzgruppen, unter Freisetzung der letztlich gewünschten Hydroxylgruppen, erfolgt ebenfalls nach Methoden, die dem Fachmann bekannt sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acetal-, Ketal- oder Ätherschutzgruppen können beispielsweise durch saure Hydrolyse abgespalten werden.

Die Umsetzung der erfindungsgemäßen Verbindungen der Formel I zu den physiologisch verträglichen Salzen mit den dem Fachmann dafür gebräuchlichen anorganischen oder organischen Basen erfolgt ebenfalls nach bekannten Methoden.

Die verfahrensgemäß eingesetzten neuen Carbonsäuren der Formel II erhält man nach an sich bekannten Methoden durch Umsetzung der bekannten 3-Amino- bzw. 3-nieder-Alkylamino-2,4,6-

trijod-benzoesäure mit einer aliphatischen Dicarbonsäure der Formel HOOC-X-COOH in Form ihres cyclischen Anhydrides

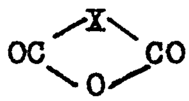

, ihres Dicarbonsäuredichlorides ClCO-X-COCl oder ihres nieder-Alkyl-esterchlorides ClCO-X-CO-O-(nieder-Alkyl), wobei X die obengenannte Bedeutung hat.

Setzt man die Dicarbonsäure HOOC-X-COOH in Form ihres cyclischen Anhydrides ein, erfolgt die Umsetzung vorzugsweise bei einer Temperatur von 80 $^{o}$C bis 120 $^{o}$C, vorzugsweise bei 100 $^{o}$C in einem polaren Lösungsmittel wie Dimethylformamid, Hexamethylphosphorsäuretriamid, vorzugsweise in Dimethylacetamid in Gegenwart einer Säure wie beispielsweise Schwefelsäure, Phosphorsäure, vorzugsweise p-Toluolsulfonsäure.
Das Rohprodukt wird nach der Umsetzung durch Wasserfällung gewonnen und über das Salz mit einer Base wie beispielsweise Natrium- oder Kaliumhydroxid, vorzugsweise Ammoniak gereinigt und anschließend die Säure der Formel II durch Ansäuern mit einer Mineralsäure wie beispielsweise Salzsäure wieder in Freiheit gesetzt, wie am folgenden Beispiel näher erläutert wird:

Diglycolsäure-mono-3-carboxy-2,4,6-trijod-N-methyl-anilid

Eine Lösung von 952 g (1,8 Mol) 3-Methylamino-2,4,6-trijod-benzoesäure in 1,8 l Dimethylacetamid wird mit 418 g (3,6 Mol) Diglycolsäureanhydrid und 90 g p-Toluolsulfonsäure versetzt und 7 Stunden auf dem Dampfbad unter Rühren erwärmt. Nach Rühren über Nacht bei Raumtemperatur wird die dunkelbraune Lösung im Vakuum weitgehend eingedampft. Der zähflüssige Rückstand wird in 18 Liter Wasser eingetragen, über Nacht gerührt, im Mörser verrieben und in 9 Liter frischem Wasser ca. eine Stunde ausgerührt. Der Niederschlag wird abgesaugt und im Vakuum bei 50°C getrocknet. Das Rohprodukt (1043 g) wird in 5,2 Liter Äthanol gelöst, blankfiltriert, mit 274 ml 13 n Ammoniak versetzt und über Nacht bei Raumtemperatur gerührt. Nach Kühlen in Eis wird das auskristallisierte Salz abgesaugt, in 10 Liter Wasser gelöst, mit 100 g Kohle gerührt, filtriert und mit 356 ml 12 n Salzsäure angesäuert. Der anfangs schmierige Niederschlag wandelt sich (nach Impfen) langsam um. Nach Rühren über das Wochenende wird der Niederschlag abgesaugt, in 4,5 Liter Wasser eine Stunde ausgerührt, abgesaugt und im Vakuum bei 50°C getrocknet. Ausbeute an Titelverbindung 730,3 g (62,9 % der Theorie), Fp.: 177°/179-181°C; Jod berechnet 59,0 %, gefunden 59,0 %.

Wird die Dicarbonsäure HOOC-X-COOH in Form ihres Dichlorides mit 3-Amino- oder 3-nieder-Alkylamino-2,4,6-trijod-benzoesäure umgesetzt, führt man die Reaktion bei 0°C bis Raumtemperatur, vorzugsweise bei 0°C in einem polaren Lösungsmittel wie Dimethylformamid, Dioxan, vorzugsweise in Dimethylacetamid durch, wobei man nach beendeter Umsetzung zunächst das Säurechlorid der allgemeinen Formel II a

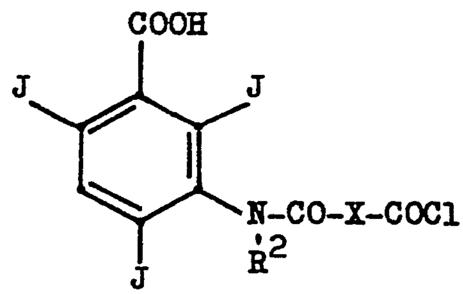

(II a)

erhält, das durch Behandlung mit wäßrigem Alkalimetallhydroxid, vorzugsweise Natriumhydroxid hydrolysiert wird.

Aus dem dabei entstandenen Alkalimetallsalz wird die Carbonsäure der Formel II durch Zusatz von Mineralsäure, vorzugsweise Salzsäure, in Freiheit gesetzt. Sie muß anschließend noch von dem als Nebenprodukt entstandenen Dicarbonsäurebisanilid der Formel II b

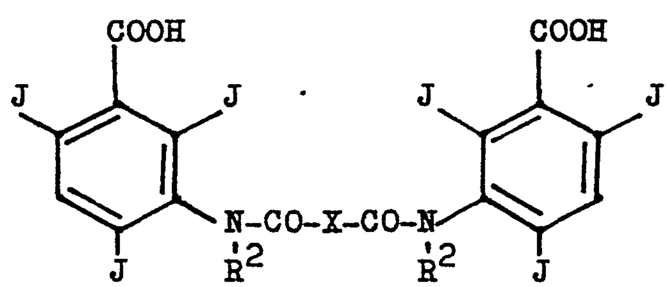

(II b)

aufgrund unterschiedlicher Löslichkeit abgetrennt werden.

Dieses Herstellungsverfahren wird durch das folgende Beispiel näher erläutert:

3 ,6 ,9 -Tri-oxaundecandisäure-mono-3-carboxy-2,4,6-trijodanilid

82 ml (400 mMol) 3,6,9-Trioxaundecandisäure-chlorid werden unter Eiskühlung in 100 ml Dimethylacetamid gelöst. In diese Lösung wird unter Eiskühlung eine Lösung von 51,5 g (100 mMol) wasserfreier 3-Amino-2,4,6-trijod-benzoesäure in 200 ml Dimethylacetamid rasch eingetragen. Nach 10 Minuten wird das Eisbad entfernt und die Lösung 48 Stunden bei Raumtemperatur gerührt. Anschließend wird das Lösungsmittel im Vakuum weitgehend abdestilliert und der ölige Rückstand in 1 Liter Wasser unter Eiskühlung mit 152 ml 11 n Natronlauge versetzt. Nach 16 Stunden wird die Lösung mit 10 g Kohle ausgerührt, filtriert, mit 8,5 Liter Wasser verdünnt und mit 175 ml 12 n Salzsäure angesäuert. Dabei fällt das als Nebenprodukt entstandene entsprechende Disäurebisanilid (etwa12 g) aus und wird abgesaugt. Das Filtrat wird im Vakuum auf ca. 600 ml eingeengt, wobei ein Niederschlag ausfällt. Er wird abgesaugt, in 100 ml Wasser im Mörser fein verrieben, eine Stunde gerührt, abgesaugt und getrocknet.

Ausbeute: 54,0 g (75,1 % der Theorie) der Titelverbindung.

Fp.: 109-113°C. Jod berechnet 53,0 %, gefunden 52,7 %.

Verwendet man für die Herstellung der Carbonsäuren II eine Dicarbonsäure HOOC-X-COOH in der Form ihres nieder-Alkyl-ester-chlorides ClCO-X-COO-(nieder-Alkyl), setzt man dieses bei einer Temperatur von 20°C bis 80°C, vorzugsweise bei 50°C, in einem polaren Lösungsmittel wie Dimethylformamid, Hexamethylphosphorsäuretriamid, vorzugsweise in Dimethylacetamid mit 3-Amino- oder 3-N-nieder-Alkylamino-2,4,6-trijodbenzoesäure um und erhält durch Wasserfällung zunächst den Monoester II c

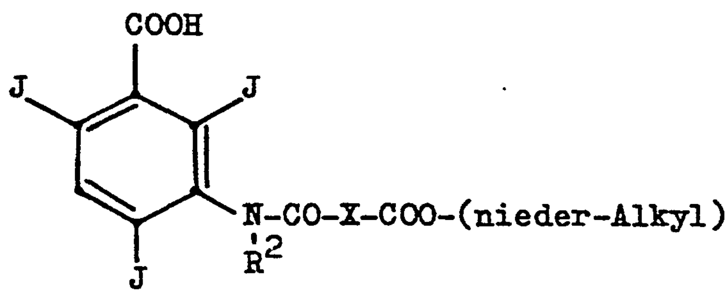 (II c) ,

der durch Behandlung mit Alkalihydroxid in Wasser mit einem Lösungsvermittler, vorzugsweise Dioxan bei 60°C bis 100°C, vorzugsweise bei 90 °C zur Säure der Formel II verseift und durch Salzbildung gereinigt wird wie in den folgenden Herstellungsbeispielen näher erläutert wird:

Glutarsäure-mono-3-carboxy-2,4,6-trijod-N-methyl-anilid

In eine Mischung von 15,9 g (30 mMol) 3-N-Methylamino-2,4,6-trijod-benzoesäure in 36 ml Dimethylacetamid werden unter Rühren 9,95 ml (72 mMol) Glutarsäure-monomethylesterchlorid innerhalb von 5 Minuten eingetropft (Wärmetönung). Nach Rühren über Nacht wird die Lösung mit 150 ml Wasser versetzt und über Nacht stehengelassen. Die Schmiere wird abgetrennt, einmal mit Wasser gewaschen, mit 100 ml Dioxan und 100 ml Wasser versetzt, unter Rühren auf 85°C erwärmt und während 45 Minuten durch portionsweise Zugabe von 11 n Natronlauge (etwa 7,5 ml) auf pH 9 gehalten. Dann wird das Dioxan im Vakuum abdestilliert, die wäßrige Lösung auf 150 ml verdünnt und mit 8,25 ml 12 n Salzsäure unter Rühren angesäuert. Nach einigen Stunden wird die Fällung abgesaugt, in 100 ml Wasser ausgerührt, abgesaugt und getrocknet.
Ausbeute: 14,6 g (75,6 % der Theorie) Titelverbindung,
Fp.: 238-239°C.

In analoger Weise erhält man

Glutarsäure-mono-3-carboxy-2,4,6-trijod-anilid, in dem man Glutarsäure-monomethylesterchlorid mit 3-Amino-2,4,6-trijod-benzoesäure umsetzt und das Endprodukt aus Eisessig umkristallisiert.
Ausbeute: 14,5 g (76,9 % der Theorie),
Fp.: 227-230°C.

Adipinsäure-mono-3-carboxy-2,4,6-trijod-N-methyl-anilid

In eine Lösung von 158,7 g (300 mMol) 3-Methylamino-2,4,6-trijod-benzoesäure in 360 ml Dimethylacetamid werden unter Rühren und leichtem Kühlen (Innentemperatur max. 50°C) 112 ml (720 mMol) Adipinsäure-monomethylesterchlorid innerhalb von 15 Minuten zugetropft. Nach Rühren über Nacht bei Raumtemperatur wird in 1,5 Liter Wasser eingetragen und von der abgeschiedenen Schmiere dekantiert. Zu Verseifung wird die Schmiere in 1,5 Liter Dioxan und 1,5 Liter Wasser auf dem Dampfbad 45 Minuten unter Rühren portionsweise mit 11 n Natronlauge (insgesamt 100 ml) versetzt, bis der pH-Wert nicht mehr unter 9 sinkt. Das Dioxan wird im Vakuum abdestilliert, die restliche Lösung mit Wasser auf 1,5 Liter verdünnt und mit 100 ml 12 n Salzsäure angesäuert. Der schmierige Niederschlag wird in frischem Wasser im Mörser verrieben, bis er weitgehend erstarrt. Dieses Rohprodukt wird in 1,8 Liter Methanol durch Zusatz von 48 ml 13 n Ammoniak gelöst, mit 18 g Kohle behandelt, filtriert, mit 1,8 Liter Wasser versetzt, im Vakuum vom Methanol befreit und mit 100 ml 12 n Salzsäure angesäuert. Nach Rühren über Nacht wird der Niederschlag bis zum völligen Erstarren unter frischem Wasser im Mörser verrieben.

Ausbeute: 170,9 g (86,7 % der Theorie) Titelverbindung, Fp.: 224-226°C.

Sebazinsäure-mono-2,4,6-trijod-3-carboxy-N-methyl-anilid

Die Lösung von 158,7 g (300 mMol) 3-Methylamino-2,4,6-trijodbenzoesäure in 360 ml Dimethylacetamid wird unter Rühren und Kühlung (Innentemperatur max. $50^{\circ}C$) innerhalb von 15 Minuten mit 169 g (720 mMol) Sebazinsäuremonomethylesterchlorid versetzt. Nach Rühren über Nacht wird die Lösung mit 150 ml Wasser versetzt und wiederum über Nacht bei Raumtemperatur stehengelassen. Die Schmiere wird in 1,5 Liter Dichloräthan unter Erwärmen auf dem Dampfbad gelöst, mehrmals mit Wasser ausgeschüttelt und anschließend zur Trockne eingedampft. Dieser Rückstand wird zur Entfernung des Sebazinsäuremonomethylesters dreimal mit je 1 Liter Hexan ausgekocht, anschließend mit je 600 ml Dioxan und Wasser versetzt, unter Rühren auf $85^{\circ}C$ erwärmt und während 45 Minuten durch portionsweise Zugabe von 11 n Natronlauge (etwa 70 ml) bei pH 9 verseift. Dann wird das Dioxan im Vakuum abdestilliert, die wäßrige Lösung auf 1,5 Liter verdünnt und mit 90 ml 12 n Salzsäure angesäuert. Die ausgefallene Schmiere wird mehrmals mit Wasser durchgearbeitet bis sie erstarrt.
Ausbeute: 209 g (97,7 % d. Theorie) Titelverbindung,
Fp.: $157-164^{\circ}C$.

In analoger Weise wird
Sebazinsäure-mono-2,4,6-trijod-3-carboxy-anilid erhalten, indem man 154,4 g (300 mMol) 3-Amino-2,4,6-trijod-benzoesäure in 360 ml Dimethylacetamid unter Rühren und Kühlen mit 169 g (720 mMol) Sebazinsäuremonomethylesterchlorid umsetzt, mit Wasser fällt, über Dichloräthan und Hexan und in Dioxan/Wasser verseift. Ausbeute: 181 g (86,3 % der Theorie), Fp.: 189-193°C.

Die verfahrensgemäß eingesetzten Anilinverbindungen der allgemeinen Formel III mit $R^1$ in der obengenannten Bedeutung sind bekannt, worin $R^3$ als $R^{3'}$ Wasserstoff oder einen gerad- oder verzweigtkettigen niederen Alkylrest bedeutet. Anilinverbindungen der allgemeinen Formel III, worin $R^3$ als $R^{3''}$ einen niederen, gerad- oder verzweigtkettigen mono- oder polyhydroxylierten Alkylrest bedeutet, wobei vorhandene Hydroxylgruppen funktionell geschützt vorliegen können, werden nach an sich bekannten Methoden hergestellt, beispielsweise indem man 3-nieder-Alkoxycarbonyl-5-nitro-benzoesäure mit einem Amin der Formel $R^{3''}-NH_2$ bei 40° - 80°C, vorzugsweise bei 60°C, durch Aminolyse umsetzt zu der entsprechenden 3-($NR^{3''}$-Carbamoyl)-5-Nitro-benzoesäure, diese Verbindung danach in an sich bekannter Weise hydriert und die so erhaltene 3-($NR^{3''}$-Carbamoyl)-5-Amino-benzoesäure in dem Fachmann bekannter Weise jodiert zu 3-($NR^{3''}$-Carbamoyl)-5-amino-2,4,6-trijod-benzoesäure, wie im nachfolgenden Beispiel näher erläutert wird.

5-Amino-N-(2-methoxyäthyl)-2,4,6-trijod-isophthalamsäure

1.) 239,2 g (1 Mol) 5-Nitroisophthalsäuremonoäthylester werden in 444 ml (5 Mol) 2-Methoxyäthylamin gelöst, wobei die Temperatur auf 60°C ansteigt. Nach 2-tägigem Rühren bei Raumtemperatur wird der Ansatz im Vakuum bei 70°C eingeengt, mit 2,4 Liter Wasser gelöst, mit Salzsäure auf pH 7 eingestellt, mit Kohle behandelt und dann mit 12 n Salzsäure auf pH 1 angesäuert. Die Fällung liefert 217,6 g (81,1 % der Theorie) 5-Nitro-N-(2-methoxyäthyl)-isophthalamsäure; Fp.: 203-207°C.

2.) Eine Lösung von 89 g (0,33 Mol) obiger Nitroverbindung in 0,7 Liter Wasser und 185 ml 2 n Ammoniak wird unter Zusatz von 7 g Raney-Nickel-Katalysator mit Wasserstoff bei 50 at Anfangsdruck hydriert. Anschließend wird die filtrierte Lösung auf 1,7 Liter verdünnt und innerhalb von 150 Minuten in eine 70-80°C warme Lösung von 1,7 Mol Chlorjod in 4 Liter 1,5 n Salzsäure getropft. Nach weiteren 2 Stunden läßt man über Nacht erkalten, saugt die Fällung ab und rührt sie mit Wasser gründlich aus. Ausbeute: 147 g (71,5 % der Theorie) Titelverbindung, Fp.: 234-238°C (Zersetzung).

Die folgenden Beispiele sollen das erfindungsgemäße Verfahren näher erläutern:

Beispiel 1

3-Oxapentandisäure-1-(3-carboxy-2,4,6-trijod-N-methyl-anilid)-5-(3-acetamido-5-carboxy-2,4,6-trijod-anilid)

In eine Lösung von 225,7 g (350 mMol) Diglycolsäure-mono-3-carboxy-2,4,6-trijod-N-methylanilid in 700 ml Dimethylacetamid werden unter Eiskühlung 55,8 ml (770 mMol) Thionylchlorid in 60 Minuten zugetropft. Das Eisbad wird nach 10 Minuten entfernt und die Lösung über Nacht bei Raumtemperatur gerührt. Anschließend werden 196 g (343 mMol) 3-Amino-5-acetamido-2,4,6-trijod-benzoesäure eingetragen; dabei Wärmetönung bis + 40°C. Nach weiteren zwei Tagen wird die Lösung in 3,5 Liter Wasser eingerührt; der ausfallende Niederschlag wird mit frischem Wasser behandelt, abgesaugt und getrocknet. Das Rohprodukt (409 g) wird mit 2 Liter Äthanol gelöst, filtriert und mit 141 ml (717 mMol) Dicyclohexylamin versetzt. Nach Rühren über Nacht und nach Kühlen in Eis wird das kristalline Salz abgesaugt und mit wenig Äthanol nachgewaschen; man erhält 245 g (46 % d. Theorie). Dieses Salz wird in 2,4 Liter Wasser suspendiert und mit 50 ml 11 n Natronlauge versetzt. Aus der trüben Lösung wird das sich abscheidende Dicyclohexylamin abgetrennt, die Lösung über ein doppeltes Faltenfilter filtriert, mit 24 g Kohle behandelt, filtriert und mit 105 ml 12 n Salzsäure angesäuert. Nach Rühren über Nacht wird der Niederschlag abgesaugt, mit 1,2 Liter Wasser ca. eine Stunde ausgerührt, abgesaugt und im Vakuum bei 50°C getrocknet. Ausbeute an Titelverbindung 175,1 g (42,6 % der Theorie), Fp.: 261-263°C (Zersetzung), Jod berechnet 63,5 %, gefunden 63,05 %.

0079397

Beispiel 2

3-Oxapentandisäure-1-(3-carboxy-2,4,6-trijod-N-methyl-anilid)-5-(3-carboxy-5-N-methylcarbamoyl-2,4,6-trijod-anilid)

In eine Lösung von 225,7 g (350 mMol) Diglycolsäure-mono-3-carboxy-2,4,6-trijod-N-methylanilid in 700 ml Dimethylacetamid werden unter Eiskühlung 55,8 ml (770 mMol) Thionylchlorid in 40 Minuten getropft. Das Eisbad wird nach 10 Minuten entfernt und die Lösung über Nacht bei Raumtemperatur gerührt. Anschließend werden 196 g (343 mMol) 2,4,6-Trijod-5-amino-isophthalsäure-monomethylamid eingetragen. Dabei Wärmetönung bis + 35°C. Am nächsten Tag wird die Lösung in 3,5 Liter Wasser eingerührt, der Niederschlag nach einiger Zeit abgesaugt, in 1,75 Liter Wasser ausgerührt, abgesaugt und im Vakuum bei 50°C getrocknet. Das Rohprodukt (572 g) wird mit 2 Liter Äthanol gelöst, filtriert und mit 182 ml (926 mMol) Dicyclohexylamin versetzt. Nach Rühren über Nacht und nach Kühlen in Eis wird das kristalline Salz abgesaugt und mit wenig Äthanol nachgewaschen. Anschließend wird es in 4 Liter Wasser suspendiert und mit 126 ml 11 n Natronlauge versetzt, wobei sich das Dicyclohexylamin abscheidet. Nach dessen Abtrennung wird die Lösung filtriert, mit Kohle behandelt und mit 255 ml 12 n Salzsäure angesäuert. Der Niederschlag wird mit Wasser ausgerührt, abgesaugt und getrocknet. Ausbeute: 291,3 g (70 % der Theorie) Titelverbindung, Fp.: 271-273°C (Zersetzung), Jod berechnet 59,0 %, gefunden 58,4 %.

Beispiel 3

3-Oxapentandisäure-1-(3-carboxy-2,4,6-trijod-N-methyl-anilid)-5-(3-carboxy-5-äthylcarbamoyl-2,4,6-trijod-anilid)

Analog Beispiel 2 wird eine Lösung von 225,7 g (350 mMol) Diglycolsäure-mono-3-carboxy-2,4,6-trijod-N-methylanilid in 700 ml Dimethylacetamid nacheinander mit 55,8 ml (770 mMol) Thionylchlorid und 201 g (343 mMol) 5-Amino-N-äthyl-2,4,6-trijod-isophthalamsäure versetzt und nach beendeter Reaktionszeit in 3,5 Liter Wasser eingerührt. Der Niederschlag wird abgesaugt, mit Wasser ausgerührt, abgesaugt und getrocknet. Dieses Rohprodukt (487 g) wird in 4,2 Liter Wasser suspendiert und durch Zusatz von 72 ml 13 n Ammoniak gelöst, mit 42 g Kohle behandelt und mit 94 ml 12 nSalzsäure angesäuert.
Die Fällung wird abgesaugt, in Wasser ausgerührt und getrocknet.
Ausbeute: 337,1 g (81,0 % der Theorie) Titelverbindung, Fp.: 240-243°C (Zersetzung), Jod berechnet 62,8 %, gefunden 63,0 %.

Beispiel 4

3-Oxapentandisäure-1-(3-carboxy-2,4,6-trijod-N-methyl-anilid)-5-(3-carboxy-5-[2-methoxyäthylcarbamoyl]-2,4,6-trijod-anilid)

Analog Beispiel 2 wird eine Lösung von 225,7 g (350 mMol) Diglycolsäure-mono-3-carboxy-2,4,6-trijod-N-methylanilid in 700 ml Dimethylacetamid nacheinander mit 55,8 ml (770 mMol) Thionylchlorid und 211 g (343 mMol) 5-Amino-N-(2-methoxyäthyl)-2,4,6-trijod-isophthalamsäure versetzt und in 3,5 Liter Wasser eingerührt. Der Niederschlag wird abgesaugt, mit Wasser ausgerührt, abgesaugt und getrocknet. Das Rohprodukt (483,5 g) wird in 4,2 Liter

Wasser suspendiert und durch Zusatz von 72 ml 13 n Ammoniak gelöst, mit 40 g Kohle behandelt und mit 94 ml 12 n Salzsäure angesäuert. Die Fällung wird abgesaugt, mit Wasser ausgerührt, abgesaugt und getrocknet. Ausbeute: 339,8 g (79,7 % der Theorie) Titelverbindung, Fp.: 240-247°C (Zersetzung), Jod berechnet 61,3 %, gefunden 61,0 %.

Beispiel 5

3-Oxapentandi-säure-1-(3-carboxy-2,4,6-trijod-N-methyl-anilid)-5-(3-carboxy-5-acetamidomethyl-2,4,6-trijod-anilid)

Analog Beispiel 1 wird eine Lösung von 6,45 g (10 mMol) Diglycolsäure-mono-3-carboxy-2,4,6-trijod-N-methylanilid in 20 ml Dimethylacetamid nacheinander mit 1,6 ml (22 mMol) Thionylchlorid und 5,86 g (10 mMol) 2,4,6-Trijod-3-amino-5-acetamidomethyl-benzoesäure versetzt, sieben Tage bei Raumtemperatur gerührt und dann in 100 ml Wasser eingerührt. Die Fällung wird abgesaugt, getrocknet (12,3 g) und aus Eisessig umkristallisiert. Ausbeute: 7,9 g (65 % der Theorie) Titelverbindung vom Fp. 231-238°C (Zersetzung), Jod berechnet 62,8 %, gefunden 61,9 %.

Beispiel 6

3,6,9-Tri-oxaundecandisäure-1-(3-carboxy-2,4,6-trijod-anilid)-11-(3-carboxy-5-methylcarbamoyl-2,4,6-trijod-anilid

In eine Lösung von 71,9 g (100 mMol) 3,6,9-Trioxaundecandisäure-mono3-carboxy-2,4,6-trijod-anilid in 200 ml Dimethylacetamid werden unter Eiskühlung 16 ml (220 mMol) Thionylchlorid getropft. Nach 6-ständigem Rühren bei Raumtemperatur wird mit 57,2 g (100 mMol) 5-Amino-2,4,6-

trijod-isophthalsäure-monomethylamid versetzt, 70 Stunden weitergerührt und schließlich in 1 Liter Wasser eingerührt. Nach 20-stündigem Rühren wird der erstarrte Niederschlag abgesaugt, mit Wasser gewaschen und getrocknet. Dieses Rohprodukt (156 g) wird in 636 ml Äthanol unter Zusatz von 54 ml Dicyclohexylamin gelöst. Nach Rühren über Nacht und Kühlen in Eis wird das auskristallisierte Salz abgesaugt, auf der Fritte mit Äthanol gewaschen, in 1,4 Liter Wasser suspendiert und mit 37 ml 11 n Natronlauge versetzt. Das Dicyclohexylamin wird abgetrennt, die wäßrige Lösung filtriert, mit 14 g Kohle behandelt und mit 75 ml 12 n Salzsäure angesäuert. Die mit Wasser ausgerührte Fällung liefert 104 g (81,5 % der Theorie) Titelverbindung vom Fp.: 199-205°C; Jod berechnet 59,8 %, gefunden 59,0 %.

<u>Beispiel 7</u>

<u>3,6,9-Trioxaundecandisäure-1-(3-carboxy-2,4,6-trijod-anilid)-11-(5-acetamido-3-carboxy-2,4,6-trijod-anilid)</u>

Analog Beispiel 6 wird eine Lösung von 71,9 g (100 mMol) 3,6,9-Trioxaundecandisäure-mono-3-carboxy-2,4,6-trijod-anilid in 200 ml Dimethylacetamid nacheinander mit 16 ml (220 mMol) Thionylchlorid und 56,06 g (98 mMol) 3-Amino-5-acetamido-2,4,6-trijod-benzoesäure umgesetzt und nach weiteren 48 Stunden in 1 Liter Wasser eingerührt, wobei ein Niederschlag entsteht. Er wird mit Wasser gewaschen, getrocknet, in 950 ml Äthanol gelöst und mit 43 ml (220 mMol) Dicyclohexylamin versetzt, worauf ein Niederschlag entsteht. Er wird scharf abgesaugt, in 1,3 Liter Wasser suspendiert und unter Rühren mit 30 ml 11 n Natronlauge versetzt, das Dicyclohexylamin wird abgetrennt, die wäßrige Lösung filtriert, mit 13 g Kohle behandelt und mit 60 ml 12 n Salzsäure angesäuert.

Ausbeute: 87,9 g (69,1 % der Theorie),
Fp. 240-248°C (Zersetzung); Jod berechnet: 59,8 %, gefunden 59,4 %.

Beispiel 8

Glutarsäure-1-(3-carboxy-2,4,6-trijod-N-methyl-anilid)-5-(3-carboxy-5-N-methylcarbamoyl-2,4,6-trijod-anilid)

Eine Lösung von 128,6 g (200 mMol) Glutarsäure-mono-3-carboxy-2,4,6-trijod-N-methylanilid in 400 ml Dimethylacetamid wird unter Eiskühlung mit 31,9 ml (440 mMol) Thionylchlorid und nach Rühren über Nacht bei Raumtemperatur mit 112,1 g (196 mMol) 2,4,6-Trijod-5-amino-isopthalsäure-mono-methylamid versetzt. Nach Rühren über Nacht wird die Lösung in 2 Liter Wasser eingetragen. Der entstandene Niederschlag wird abgesaugt und getrocknet. Dieses Rohprodukt (248 g) wird in 2,3 Liter Wasser durch Zusatz von 33 ml 13 n Ammoniak gelöst, mit 23 g Kohle behandelt und durch Zusatz von 50 ml 12 n Salzsäure ausgefällt. Der Niederschlag wird abgesaugt, in 2 Liter 1 n Salzsäure und Wasser ausgerührt, scharf abgesaugt und im Vakuum bei 60°C im Luftstrom getrocknet.

Ausbeute: 191,5 g (81,3 % der Theorie) Titelverbindung, Fp.: 257-261°C (Zersetzung); Jod berechnet 68,6 %, gefunden 63,1 %.

Beispiel 9

Hexandisäure-1-(3-carboxy-2,4,6-trijod-N-methyl-anilid)-6-(3-carboxy-5-N-methylcarbamoyl-2,4,6-trijod-anilid)

Eine Lösung von 13,1 g (20 mMol) Adipinsäure-mono-3-carboxy-2,4,6-trijod-N-methylanilid in 40 ml Dimethylacetamid wird unter Eiskühlung mit 3,2 ml (44 mMol) Thionylchlorid und nach Rühren über Nacht bei Raumtemperatur mit 11,2 g (19,6 mMol) 2,4,6-Trijod-5-amino-isophthalsäure-monomethylamid versetzt. Nach Rühren über Nacht wird die Lösung in 200 ml Wasser eingerührt, wobei ein Niederschlag ausfällt. Er wird noch feucht in 120 ml Äthanol gelöst, mit 9,4 ml (48 mMol) Dicyclohexylamin versetzt und über Nacht gerührt. Das abgeschiedene

Salz wird abgesaugt, in 250 ml Wasser suspendiert und mit 6,5 ml 11 n Natronlauge versetzt. Das sich abscheidende Dicyclohexylamin wird abgetrennt. Die wäßrige Lösung wird filtriert, mit Kohle behandelt und mit 13 ml 12 n Salzsäure angesäuert. Nach Rühren über Nacht wird der Niederschlag abgesaugt, gewaschen und getrocknet.
Ausbeute: 18,1 g (91,6 % der Theorie) Titelverbindung, Fp.: 240-247°C (Zersetzung); Jod berechnet 62,9 %, gefunden 62,2 %.

Beispiel 10

Hexandisäure-1-(3-carboxy-2,4,6-trijod-anilid)-6-(3-carboxy-5-N-methylcarbamoyl-2,4,6-trijod-anilid)

Eine Lösung von 128,6 g (200 mMol) Adipinsäure-mono-2,4,6-trijod-3-carboxy-anilid in 400 ml Dimethylacetamid wird unter Eiskühlung mit 31,9 ml (440 mMol) Thionylchlorid und nach Rühren über Nacht bei Raumtemperatur mit 112,1 g (196 mMol) 2,4,6-Trijod-5-amino-isophthalsäure-monomethylamid versetzt. Wärmetönung bis ca. 35°C. Nach Rühren über Nacht wird die Lösung in 2 Liter Wasser einge-tragen, wobei ein Niederschlag ausfällt. Er wird noch feucht in 2,4 Liter Methanol gelöst, durch Zugabe von 33 ml 13 n Ammoniak auf pH 7,5 eingestellt, mit 23 g Kohle behandelt, mit 2,4 Liter Wasser verdünnt, erneut mit 23 g Kohle behandelt, auf 2 Liter eingeengt und mit 50 ml 12 n Salzsäure angesäuert. Der Niederschlag wird nach einiger Zeit abgesaugt, in 2 Liter 1 n Salzsäure ausgerührt, scharf abgesaugt und im Vakuum bei 60°C im Luftstrom getrocknet.
Ausbeute: 184,2 g (78,5 % der Theorie) Titelverbindung.

Fp.: 270-282°C (Zersetzung).
Jod berechnet 63,6 %, gefunden 64,1 %.

Beispiel 11

Herstellung einer gebrauchsfertigen Megluminsalzlösung mit 300 mg J/ml:

| | | | |
|---|---|---|---|
| 3-Oxapentandisäure-1-(3-carboxy-2,4,6-trijod-N-methylanilid)-5-(3-carboxy-5-N-methylcarbamoyl-2,4,6-trijod-anilid) | | 472,3 | g |
| N-Methylglucamin | | 153,8 | g |
| Calciumdinatriumedetat | | 0,1 | g |
| Aqua bidestillata | ad | 1000 | ml |

In etwa 550 ml vorgelegtes Aqua bidestillata werden nacheinander das Edetat, die Jodverbindung und schließlich soviel Meglumin eingetragen, daß die Lösung einen pH von 7,0 ± 0,5 erreicht. Anschließend wird die Lösung mit Aquabidestillata auf 1000 ml aufgefüllt, in Flaschen oder Ampullen abgefüllt und bei 120°C sterilisiert.

Beispiel 12

Herstellung einer gebrauchsfertigen Methylglucaminsalzlösung mit 180 mg Jod/ml:

| | | | |
|---|---|---|---|
| 3,6,9-Trioxaundecandisäure-1-(3-carboxy-2,4,6-trijod-anilid)-11-(5-acetamido-3-carboxy-2,4,6-trijod-anilid) | | 300,9 | g |
| N-Methylglucamin | | 92,3 | g |
| Calciumdinatriumedetat | | 0,1 | g |
| Aqua bidestillata | ad | 1000 | ml |

Die Lösung wird wie in Beispiel 11 hergestellt, abgefüllt und sterilisiert.

Beispiel 13

Decandisäure-1-(3-carboxy-2,4,6-trijod-N-methyl-anilid)-10-(3-carboxy-5-N-methylcarbamoyl-2,4,6-trijodanilid)

In eine Lösung von 14,3 g (20 mMol) Sebazinsäure-mono-2,4,6-trijod-3-carboxy-N-methylanilid in 40 ml Dimethylacetamid werden unter Eiskühlung 3,2 ml (44 mMol) Thionylchlorid innerhalb von 5 Minuten zugetropft. Das Eisbad wird nach 10 Minuten entfernt und die Lösung über Nacht bei Raumtemperatur gerührt. Anschließend werden 11,2 g (19,6 mMol) 2,4,6-Trijod-5-amino-isophthalsäure-monomethylamid eingetragen; dabei leichte Wärmetönung. Nach 20 Stunden wird die braune Lösung in 200 ml Wasser eingerührt. Der Niederschlag wird nach einiger Zeit abgesaugt, mit 200 ml Wasser ausgerührt, abgesaugt und getrocknet. Dieses Rohprodukt (26 g) wird in 130 ml Äthanol gelöst, mit 10,9 ml (55,6 mMol) Dicyclohexylamin versetzt und über Nacht gerührt. Das auskristallisierte Salz wird abgesaugt, mit frischem Äthanol nachgespült, in 260 ml Wasser suspendiert und mit 7,6 ml (83,5 mMol) 11 n Natronlauge versetzt. Das Dicyclohexylamin wird abgetrennt (6,2 ml), die wäßrige Lösung über ein feuchtes doppeltes Faltenfilter filtriert, mit Kohle behandelt und mit 15,3 ml (184 mMol) 12 n Salzsäure angesäuert. Nach Rühren über Nacht wird der Niederschlag abgesaugt, mit 240 ml Wasser ausgerührt und getrocknet. Ausbeute: 15,2 g (61,2 % der Titelverbindung), Fp.: ab 207°C (Zersetzung); Jod berechnet 60,1 %, gefunden 59,6 %.

Beispiel 14

Decandisäure-1-(3-carboxy-2,4,6-trijod-anilid)-10-(3-carboxy-5-N-methylcarbamoyl-2,4,6-trijodanilid)

Analog Beispiel 3 wird eine unvollständige Lösung von 14,0 g (20 mMol) Sebazinsäure-mono-2,4,6-trijod-3-carboxy-anilid in 40 ml Dimethylacetamid mit 3,2 ml (44 mMol) Thionylchlorid und anschließend mit 11,2 g (19,6 mMol) 2,4,6-Trijod-5-amino-isophthalsäure-monomethylamid umgesetzt und aufgearbeitet. Nach der Reinigung über das Dicyclohexylaminsalz erhält man 15,8 g (83,5 % der Theorie) Titelverbindung, Fp. 230-240°C (Zersetzung).

## Patentansprüche

1.) Unsymmetrisch substituierte Dicarbonsäure-bis-(2,4,6-trijodanilide) der Formel I

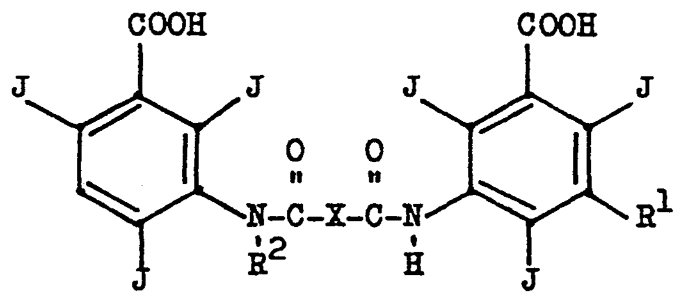

(I),

worin

X ein gerad- oder verzweigtkettiges Alkylen, das durch ein oder mehrere Sauerstoffatome unterbrochen ist,

$R^1$ den Rest -NHAcyl, $-CH_2NHAcyl$ oder $-CONHR^3$ bedeutet, wobei $R^3$ Wasserstoff oder einen niederen, gerad- oder verzweigtkettigen, gegebenenfalls mono- oder polyhydroxylierten Alkylrest darstellt und vorhandene Hydroxylgruppen funktionell abgewandelt vorliegen können

und

$R^2$ Wasserstoff oder einen niederen Alkylrest bedeutet,

sowie deren physiologisch verträgliche Salze mit anorganischen oder organischen Basen.

2.) Hexandisäure-1-(3-carboxy-2,4,6-trijodanilid)-6-(3-carboxy-5-N-methyl-carbamoyl-2,4,6-trijodanilid).

3.) 3-Oxapentandisäure-1-(3-carboxy-2,4,6-trijod-N-methylanilid)-5-(3-carboxy-5-N-methylcarbamoyl-2,4,6-trijodanilid).

4.) 3,6,9-Trioxaundecandisäure-1-(3-carboxy-2,4,6-trijodanilid)-11-(5-acetamido-3-carboxy-2,4,6-trijodanilid).

5.) Verfahren zur Herstellung unsymmetrisch substituierter Dicarbonsäure-bis-(2,4,6-trijodanilide) der Formel I, dadurch gekennzeichnet, daß man in an sich bekannter Weise eine Verbindung der allgemeinen Formel II

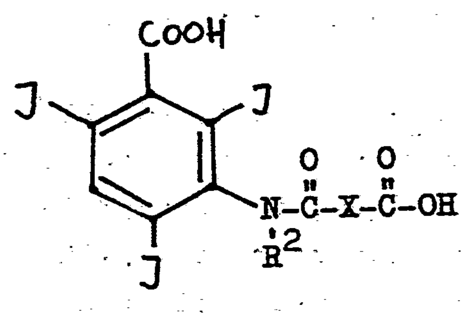

(II) ,

worin X und $R^2$ die obengenannte Bedeutung hat, mit einem Chlorierungsreagenz und einer trijodierten Aminobenzoesäure der Formel III

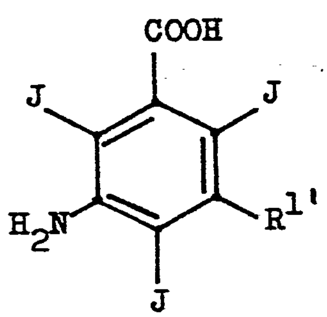

(III) ,

worin $R^{1'}$ die obengenannte Bedeutung für $R^1$ hat, jedoch gegebenenfalls vorhandene freie Hydroxylgruppen in funktionell abgewandelter Form vorliegen, umsetzt, gegebenenfalls anschließend Schutzgruppen abspaltet und durch Umsetzung mit anorganischen oder organischen Basen Salze herstellt.

6.) Röntgenkontrastmittel auf Basis von Verbindungen gemäß Anspruch 1 bis 4.

7.) 2,4,6-Trijodphenyl-dicarbonsäuren der Formel II

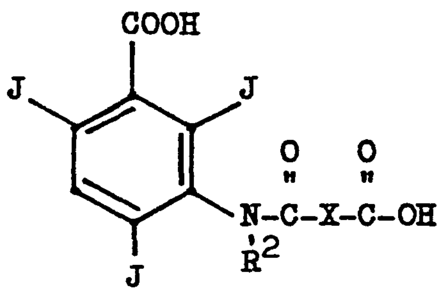

(II ) ,

worin X und $R^2$ die obengenannte Bedeutung haben.

8.) Verfahren nach Anspruch 5, dadurch gekennzeichnet, daß man die Verbindungen der Ansprüche 2 bis 4 herstellt.

Europäisches Patentamt

# EUROPÄISCHER RECHERCHENBERICHT

0079397

Nummer der Anmeldung

EP 81 10 9668.4

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| A | EP - A1 - 0 011 849 (SCHERING AG)<br>* Zusammenfassung *<br>-- | | C 07 C 103/46<br>A 61 K 49/04 |
| A | DE - A - 2 141 803 (SCHERING AG)<br>* Ansprüche *<br>---- | | |

RECHERCHIERTE SACHGEBIETE (Int. Cl.3)

A 61 K 49/04
C 07 C 103/46

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 18-06-1982 | BREW |